# EUROPÄISCHE PATENTSCHRIFT

(11) **EP 1 171 045 B1**
(45) Veröffentlichungstag und Bekanntmachung des Hinweises auf die Patenterteilung: **22.11.2006**
(21) Anmeldenummer: 01909726.0
(22) Anmeldetag: 08.02.2001
(51) Int. Cl.: A61B 17/34

(54) **TROKARHÜLSE MIT VERÄNDERLICHER DICHTUNGSÖFFNUNG**
TROCAR SLEEVE WITH VARIABLE SEAL OPENING
TROCART POURVU D'UNE OUVERTURE DE JOINT MODIFIABLE

(30) Priorität: 26.02.2000 DE 10009132
(43) Veröffentlichungstag der Anmeldung: 16.01.2002
(73) Patentinhaber: Karl Storz GmbH & Co., 78532 Tuttlingen (DE)
(72) Erfinder: DITTRICH, Horst, 78194 Immendingen (DE); OBERLÄNDER, Martin, 78532 Tuttlingen (DE); SAUER, Michael, 78532 Tuttlingen (DE)
(74) Vertreter: Weller, Wolfgang
(86) Internationale Anmeldenummer: PCT/EP2001/001351
(87) Internationale Veröffentlichungsnummer: WO 2001/062167

(56) Entgegenhaltungen:
- EP-A- 0 834 279
- US-A- 5 209 737
- US-A- 5 458 640
- US-A- 5 657 963
- US-A- 5 779 697

## Beschreibung

Die Erfindung betrifft eine Trokarhülse, mit einem proximalseitig angeordneten Gehäuse, von dem distalseitig ein Schaft vorspringt, und mit einer Dichtung aus elastischem Material, um ein in die Trokarhülse eingeschobenes Instrument gasdicht nach proximal abzudichten, wobei die Dichtung eine mittige Öffnung aufweist, deren Durchmesser veränderbar ist, wobei die Dichtung eine Ruhelage aufweist, in der die mittige Öffnung einen kleinsten Durchmesser aufweist, und mit einer Spreizvorrichtung, die in die Dichtung einfahrbar ist, diese dabei dehnt, wodurch der Durchmesser der mittigen Öffnung vergrößerbar ist, wobei die Spreizvorrichtung eine axial verschiebbare Hülse aufweist, deren eine ringförmige Stirnfläche in die Dichtung einfahrbar ist.

Eine derartige Trokarhülse ist aus der US-A-5 657 963 bekannt.

Die Bewegung der axial verschiebbaren Hülse der Spreizvorrichtung geschieht dadurch, daß die Hülse mit zwei diametral gegenüberliegenden, elastischen Elementen im Gehäuse abgestützt ist. Eines der Elemente ist über einen Hebel zur Außenseite hin verlängert. Mittels dieses vorspringenden Hebels kann durch Bewegen desselben über die elastischen Elemente die Hülse nach distal verschoben werden, wobei die Dichtung gespreizt wird. Der Hebel wird dabei zwischen einer "geschlossenen" und "offenen" Position hin- und herbewegt.

In der "offenen" Position ist die Dichtung maximal gespreizt. Wird der Hebel in dieser Position freigegeben, wird die Hülse auf Grund der Rückstellkraft der geweiteten elastischen Dichtung wieder in die "geschlossene" Position zurückbewegt. Dadurch, daß die Hülse mit den diametral gegenüberliegenden, elastischen Elementen im Gehäuse verspannt ist, entsteht ein bistabiles System, das zwischen zwei Endpositionen in einer Art Flip-Flop-Schaltung hin- und herbewegbar ist.

Trokare dienen dazu, um für die minimal-invasive Chirurgie eine Inzision im Körper zu bewerkstelligen, durch die Instrumente von der Außenseite in den Körper eingeführt werden können. Ein Trokar besteht aus einer Trokarhülse und, zum Setzen des Trokares, aus einem Trokardorn mit in den meisten Fällen angeschärfter Spitze. Nach Setzen des Trokars wird der Trokardorn abgezogen, und die Trokarhülse verbleibt in der Inzision im Körper. Durch die Trokarhülse werden bei minimal-invasiven Eingriffen unterschiedliche Instrumente hindurchgeführt, beispielsweise Endoskope, medizinische Zangen oder sonstige mit einem langen Schaft versehene Instrumente. Bei dieser Operationstechnik ist es ferner üblich, über den Trokar ein Gas in die Körperhöhle zuzuführen, um diese zu insufflieren. Dazu ist im Bereich des Gehäuses der Trokarhülse meist ein seitlich vorstehender Gasanschluß mit einem Hahn vorgesehen.

Wie zuvor erwähnt, werden im Laufe eines minimal-invasiven Eingriffes zahlreiche unterschiedliche Instrumente durch die Trokarhülse eingeführt bzw. abgezogen.

Um einen Gasaustritt aus dem Körper zu verhindern, ist entweder ein Klappenventil vorgesehen, das automatisch die Trokarhülse nach proximal gasdicht schließt, wenn kein Instrument in die Hülse eingeschoben ist, oder es ist eine Dichtung in Form einer Schlitzdichtung vorgesehen, meist am Übergang zwischen Gehäuse und Schaft, die vom Instrument durchstoßen und aufgeweitet werden kann. Diese Dichtung kann zwar für einen gasdichten Abschluß sorgen, wenn kein Instrument eingeschoben ist, aufgrund der Schlitzgeometrie ist aber ein gasdichter Abschluß gegenüber dem Schaft des eingeführten Instrumentes mit meist kreisrundem Querschnitt nicht möglich.

Daher ist in der Trokarhülse eine Dichtung vorgesehen, die eine mittige Öffnung aufweist, deren Durchmesser in etwa dem Außendurchmesser des Schaftes des eingeschobenen Instrumentes entspricht.

Anders ausgedrückt, die Dichtung aus elastischem Material weist eine solche Öffnung auf, daß gerade das Instrument durchgeschoben werden kann und dennoch ein gasdichter Abschluß gewährleistet ist.

Wie eingangs erwähnt, ist es aber vorgesehen, daß Instrumente mit unterschiedlichem Schaftdurchmesser durch die Trokarhülse hindurchgeschoben werden.

Nimmt man einmal als Beispiel eine Trokarhülse mit einem lichten Innendurchmesser von 11 mm heran, so werden üblicherweise Instrumente mit einem Schaftaußendurchmesser von 5 bis 10 mm durch die Trokarhülse hindurchgeschoben.

Weist die Dichtung eine Öffnung mit einem Durchmesser von knapp 5 mm auf, könnte ein Instrument mit einem Schaftdurchmesser von 6 mm unter Überwindung eines Widerstandes gerade noch durch die Dichtung hindurchbewegt werden.

Soll aber durch diese Dichtung ein Instrument mit einem Schaftdurchmesser von 10 mm hindurchgeschoben werden, ist dies, wenn überhaupt, nur unter einem erheblichen Kraftaufwand möglich. Selbst wenn man es geschafft haben sollte, ein Instrument mit 10 mm Schaftdurchmesser durch die 5 mm große Öffnung der Dichtung hindurchzudrücken, wäre es für den Operateur äußerst störend und schwierig, während des Eingriffes das Instrument axial hin- und herzuverschieben. Dies wird allenfalls ruckweise gelingen, was nicht erwünscht ist.

In der DE 43 12 147 A1 ist vorgesehen, unterschiedliche Dichtungen mit unterschiedlichen Öffnungsdurchmessern vorzusehen, die, je nachdem, welchen Schaftdurchmesser das einzuführende Instrument hat, in die Trokarhülse eingesetzt werden. Dies erfordert baulich sehr aufwendige Maßnahmen, wie das beispielsweise aus den Ausführungsbeispielen von Fig. 9 bis 18 dieser Druckschrift ersichtlich ist. D.h. die zahlreichen Dichtungen mit den unterschiedlichen Durchmessern müssen irgendwo an der Trokarhülse befestigt sein, und über Schwenk- oder Verdrehmechanismen müssen diese dann in den mittig durchgehenden Hohlkanal der Trokarhülse eingeschwenkt oder eingedreht werden können.

Bei einem weiteren, in dieser Druckschrift beschriebenen Ausführungsbeispiel, nämlich dem in den Fig. 22a bis 23b gezeigten Ausführungsbeispiel, wird daher eine Dichtung geschaffen, deren Durchmesser von einem Minimaldurchmesser, der nahe bei 0 liegt, bis zu einem Maximaldurchmesser kontinuierlich verändert werden kann.

Dazu ist vorgesehen, ein Schlauchstück aus einem elastischen Material zwischen zwei axial voneinander beabstandeten, relativ zueinander verdrehbaren Ringen einzuspannen. Wird der eine Ring gegenüber dem anderen Ring verdreht, entsteht eine mehr oder weniger genau definierte Einschnürung auf mittiger Höhe des Schlauchstückes. Über den Umfang der beiden Ringe verteilte, diese axial längs mehrerer Mantellinien verbindende Gummistränge sorgen für eine Rückstellkraft, um das verdrillte und eingeschnürte Schlauchstück wieder in die hohlzylindrische Form zurückzudrehen.

Nachteilig an dieser Ausgestaltung ist, daß das schlauchförmige Dichtungsmaterial erheblichen Verformungen unter Ausbildung von Knickstellen und Falten ausgesetzt ist, so daß nicht nur eine erhebliche Belastung des Dichtungsmaterials vorherrscht, sondern daß durch die Faltenbildung ein einwandfreier gasdichter Abschluß zur glatten Außenseite eines eingeschobenen Instrumentes nicht gewährleistet ist. Ferner nachteilig ist, daß durch die Rückstellkraft der Gummibänder die Dichtung in Richtung Offenstellung vorgespannt ist, also konstruktionsbedingt die Tendenz aufweist, den jeweils maximalen Durchmesser der Öffnung einzunehmen. Soll also ein Instrument mit kleinerem Schaftdurchmesser eingeschoben werden, muß die Vorrichtung Maßnahmen vorsehen, um die Dichtung, entgegen der Rückstellkraft der Bänder, mit einem gewissen Dichtungsdruck an die Außenseite des Schaftes anzulegen.

Aus der US-A-5 458 640 ist eine Trokarhülse mit einem proximalseitig angeordneten Gehäuse bekannt, in dem eine Dichtung aus elastischem Material aufgenommen ist. Die Dichtung ist topfartig ausgebildet und weist bodenseitig zwei konvergierende Lippen auf, die längs eines diametral verlaufenden Schlitzes dichtend und geschlossen aneinander liegen. Zum Spreizen der aneinander liegenden Lippen ist eine Spreizvorrichtung vorgesehen, die eine axial verschiebbare Hülse aufweist.

Es ist Aufgabe der vorliegenden Erfindung, eine Trokarhülse mit einer Dichtung mit veränderlichem Öffnungsdurchmesser der eingangs genannten Art dahingehend weiterzubilden, daß der Spreizvorgang gezielt durchführbar ist und eine sichere Abdichtung bei unterschiedlichen Instrumentendurchmessern gewährleistet ist.

Erfindungsgemäß wird die Aufgabe dadurch gelöst, daß die Spreizvorrichtung in unterschiedlichen Einfahrstellungen feststellbar ist.

Diese Maßnahme hat den erheblichen Vorteil, daß die Öffnung der Dichtung auf bestimmte Durchmessergrößen erweitert wird und dann in diesem Dehnungs- oder Aufweitzustand durch die festgestellte Spreizvorrichtung gehalten wird.

Diese Maßnahme wirkt sich dann immer vorteilhafter aus, je mehr die Öffnung vergrößert wird. Um auf das mehrfach genannte Beispiel zurückzugreifen, nämlich eine Dichtung, die in der Ruhelage, also der entspannten Lage, einen Öffnungsdurchmesser von 5 mm aufweist, so wird die Rückstellkraft nach einem Spreizen auf einen Durchmesser von 6 mm noch relativ gering sein und ermöglicht dem Operateur, ein Instrument mit einem solchen Durchmesser noch zur Korrektur des axialen Sitzes in der gespreizten Dichtung hin- und herzuschieben. Ist die Öffnung allerdings auf 10 mm Durchmesser gespreizt, wird die Rückstellkraft wesentlich stärker sein, und ein axiales Verschieben wird nur unter Überwindung eines erheblichen Reibungswiderstands möglich sein.

Wird die Spreizvorrichtung nun in einer Einfahrstellung festgestellt, die gerade eine Aufweitung der Öffnung auf 10 mm verursacht hat, kann dann das Instrument in der Dichtung axial hin- und herverschoben werden. Es ist lediglich dafür Sorge zu tragen, daß der Anpreßdruck ausreichend ist, um einen gasdichten Abschluß zu schaffen. Dies kann beispielsweise dadurch erreicht werden, daß die Dichtung nur auf 9,95 mm Durchmesser gespreizt wird.

In der Ruhelage ist die Dichtung entspannt, und die Öffnung kann bspw. einen Durchmesser aufweisen, der dem Außendurchmesser eines kleinsten Schaftdurchmessers entspricht, der durch die Trokarhülse hindurchgeführt werden soll.

Soll nun ein Instrument mit einem größeren Schaftdurchmesser eingeführt werden, wird die Spreizvorrichtung betätigt, die in die Dichtung einfährt und diese dabei dehnt, wodurch der Durchmesser der mittigen Öffnung vergrößert wird. Dabei wird der Spreizvorgang durch die Spreizvorrichtung und nicht durch das Instrument selbst durchgeführt, so daß der Spreizvorgang vor Einführen des Instrumentes durchgeführt werden kann.

Zum Einführen ist es bspw. möglich, die Öffnung sogar ein klein wenig weiter aufzudehnen als notwendig, so daß das Instrument quasi berührungslos und somit einfach und widerstandsarm durch die Trokarhülse in den Körper eingeführt werden kann. Aufgrund der Elastizität des Materials entsteht beim Dehnen eine Rückstellkraft, die dazu führt, daß die Dichtung wieder in die Ruhelage zurückgeführt wird. Wird also die Spreizvorrichtung wieder freigegeben, verengt sich die Öffnung, und das Dichtungsmaterial legt sich mit dem erforderlichen Dichtungsdruck um die schaftförmige Außenseite des zwischenzeitlich eingeschobenen Instrumentes an. Dadurch ist der notwendige gasdichte Anpreßdruck gewährleistet.

Durch die ringförmige Stirnfläche der verschiebbaren Hülse wirkt die Dehnkraft gleichförmig rund um die Öffnung auf die Dichtung ein, was dann gleichzeitig entsprechend radiale Spreizkräfte verursacht, um die kreisförmige Öffnung gleichmäßig, also unter Beibehaltung dieser Kreisgeometrie, zu vergrößern. Die Hülse kann raumsparend in das üblicherweise rohrförmige Gehäuse der Trokarhülse eingesetzt werden und läßt den notwendigen mittigen Raum frei, um Instrumente oder auch den Trokardorn oder Opturatoren durchzuführen.

Zum Abziehen des Instrumentes kann dann die Spreizvorrichtung wieder etwas eingefahren werden, so daß dann das Instrument einfach, d.h. ohne Reibungswiderstand abgezogen werden kann.

Soll die axiale Einschubtiefe des Instrumentes verändert werden, kann die Spreizvorrichtung so weit eingefahren werden, daß das Instrument verschoben werden kann, ohne daß ein bemerkenswerter Gasaustritt erfolgt. Somit ist die Handhabung des Instruments zusätzlich erleichtert.

In einer weiteren Ausgestaltung der Erfindung weist die Dichtung einen sich quer zur Trokarhülsenachse erstreckenden scheibenförmigen Abschnitt auf, in dem mittig die Öffnung vorgesehen ist.

Diese Maßnahme hat den Vorteil, daß die Dichtung in ihrer Ruhelage wenig Bauraum erfordert und somit auch einfach und kostengünstig herstellbar ist. Diese Geometrie erlaubt dann beim axialen Dehnen ein gleichmäßiges Aufweiten der Öffnung unter Beibehaltung der Kreisgeometrie.

In einer weiteren Ausgestaltung ist dazu vorgesehen, daß die ringförmige Stirnfläche der Hülse koaxial zu einer kreisrunden Öffnung im scheibenförmigen Abschnitt der Dichtung angeordnet ist.

Diese geometrische Ausgestaltung sorgt zum einen für eine schonende Kraftübertragung zum Dehnen der Dichtung und stellt eine Vergrößerung der kreisrunden Öffnung unter Beibehaltung der Kreisgeometrie sicher.

In einer weiteren Ausgestaltung der Erfindung weist die Dichtung einen topfförmigen Körper auf, in dessen Boden die Öffnung vorgesehen ist, und die Hülse ist in den Körper einfahrbar und dehnt diesen axial.

Diese Maßnahme hat den Vorteil, daß die Topfgeometrie den Verformungsvorgang der Dichtung beim Dehnen schon im Ansatz ausgebildet hat, d.h. der Topf wird, wie in einer Art Tiefziehvorgang, zu einem tiefen Napf gedehnt, dessen Boden dann Material zur Verfügung stellt, um die längere Topfwand zu bilden, wobei sich die mittige Öffnung im Topf aufweitet bzw. vergrößert. Die Topfgeometrie begünstigt dabei die Beibehaltung der Kreisgeometrie beim Vergrößern der Öffnung.

In einer weiteren Ausgestaltung der Erfindung sind Dichtung und Hülse im Gehäuse angeordnet.

Diese Maßnahme hat den Vorteil, daß diese Bauteile der Spreizvorrichtung geschützt im Innern der Trokarhülse untergebracht sind und auch baulich keine raumergreifenden Maßnahmen erfordern, so daß bei schlanker Bauweise der Trokarhülse ein maximaler lichter Innenraum zum Ein- und Durchführen von Instrumenten zur Verfügung steht.

In einer weiteren Ausgestaltung der Erfindung ist eine Führung vorgesehen, durch die die Verschiebebewegung der Spreizvorrichtung führbar ist, und es sind Rastmittel vorgesehen, um die Spreizvorrichtung in bestimmten Verschiebestellungen zu verrasten.

Diese Maßnahme hat den Vorteil, daß durch die Führung eindeutig vorgegebene Verschiebebewegungen resultieren, die vom Operateur ohne besondere Aufmerksamkeit rasch durchgeführt werden können und jeweils von einer Verraststellung zur anderen einfach durchgeführt werden können, so daß dann ganz definierte Dichtungsöffnungsgrößen erzielt werden können und auch zwischen diesen variiert werden kann.

In einer weiteren Ausgestaltung der Erfindung ist die Führung eine Kulissenführung, längs der ein Kulissenelement führbar ist, das zwangsweise der Führung folgt.

Diese Maßnahme hat den Vorteil, daß durch konstruktiv einfache Maßnahmen diese Zwangsführung bewerkstelligt wird.

In einer weiteren Ausgestaltung der Erfindung ist die Kulissenführung als Herz-Kurve ausgebildet, so daß die Spreizvorrichtung zwischen einer Null-Stellung, ohne Spreizung der Dichtung, einer maximalen Spreizstellung und einer dazwischen liegenden Spreizstellung bewegbar ist.

Diese Maßnahme hat nun den erheblichen handhabungsmäßigen Vorteil, daß aus der Null-Stellung durch Verfahren längs der Herz-Kurve die Vorrichtung zunächst einmal in die maximale Spreizstellung gebracht werden kann, so daß dann ein Instrument nahezu berührungslos durch die Öffnung hindurchgeschoben werden kann. Anschließend wird die Dichtung in die dazwischen liegende Spreizstellung bewegt, eine Spreizstellung, die gerade für das eingeschobene Instrument optimal ist. Im nächsten Bewegungszyklus wird dann wieder aus dieser dazwischen liegenden Stellung auf die maximale Spreizstellung gespreizt, nämlich um das Instrument wieder abzuziehen. Im nächsten Zyklus bewegt sich dann die Spreizvorrichtung von der maximalen Spreizstellung wieder in die Null-Stellung, d.h. die Dichtung wird wieder entspannt.

Um dies anhand eines Zahlenbeispiels zu verdeutlichen, wird auf das bereits mehrfach angesprochene Zahlenbeispiel zurückgegriffen. Hat die Dichtung ausgänglich, also in der Null-Stellung, einen Öffnungsdurchmesser von 5 mm, und soll ein Instrument mit einem Schaftdurchmesser von 10 mm eingebracht werden, kann die Öffnung zunächst auf einen Maximaldurchmesser von 11 mm aufgeweitet werden. Durch diese etwas im Übermaß aufgeweitete Öffnung kann das Instrument reibungsarm durch die Dichtung hindurchgeführt und axial in die entsprechende Einschubposition gebracht werden. Im zweiten Bewegungszyklus längs der Herz-Kurve wird die Dichtung nun von einem Durchmesser von 11 mm auf 10 mm verjüngt, liegt also exakt passend an dem Schaft des Instrumentes mit 10 mm Außendurchmesser an. Der Anpreßdruck ist ausreichend, um einen gasdichten Abschluß zu gewährleisten, dennoch kann das Instrument noch axial etwas hin- und herbewegt werden. Soll das Instrument nun wieder abgenommen werden, wird durch Weiterfahren in der Herz-Kurve die Öffnung zunächst einmal wieder von 10 mm auf 11 mm erweitert, dann kann das Instrument wieder einfach abgezogen werden. Im vierten Bewegungszyklus wird dann die Vorrichtung von der maximalen Spreizstellung (11 mm) wieder in die Ausgangsposition zurückgefahren, so daß dann die Dichtung wieder ihre entspannte Lage, also die Null-Stellung mit 5 mm Öffnungsdurchmesser, einnimmt.

In einer weiteren Ausgestaltung der Erfindung weist die Spreizvorrichtung ein Betätigungsorgan auf, das von der Außenseite der Trokarhülse her betätigbar ist.

Diese Maßnahme hat den Vorteil, daß das Betätigungsorgan für die Bedienungsperson von der Außenseite ergreifbar ist, alle weiteren Bauteile aber dann innen in der Trokarhülse geschützt angeordnet werden können.

In einer weiteren Ausgestaltung der Erfindung ist das Betätigungsorgan als ein um eine quer zur Trokarhülsenachse verlaufende Achse verschwenkbarer Hebel ausgebildet.

Diese konstruktive Anordnung ermöglicht es der Bedienungsperson, beispielsweise einen Finger oder einen Daumen an den Hebel anzulegen, diesen in axialer Richtung zu drücken und dadurch die Spreizvorrichtung zu bewegen. Diese Bewegungsrichtung ist nicht nur für die Bedienungsperson ergonomisch sondern vermeidet auch die unerwünschten Drehbewegungen einer Trokarhülse in der Inzision.

In einer weiteren Ausgestaltung der Erfindung springt vom Hebel ein Stift vor, der in der Kulissenführung läuft.

Diese Maßnahme hat den Vorteil, daß durch konstruktiv einfache Maßnahmen die zuvor beschriebene Zwangsführung bewerkstelligt werden kann.

In einer weiteren Ausgestaltung der Erfindung ist der Stift federbelastet.

Diese Maßnahme hat den Vorteil, daß über die Federbelastung der Stift exakt sicher in der Kulissenführung geführt bzw. in diese hineingedrückt wird, so daß beispielsweise die Zwangsführung in der Herz-Kurve durch entsprechende Schrägen unterstützt werden kann, denen der federbelastete Stift exakt folgt.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand eines ausgewählten Ausführungsbeispiels näher beschrieben und erläutert. Es zeigen:
- Fig. 1: eine perspektivische Ansicht einer Trokarhülse mit einer erfindungsgemäßen Spreizvorrichtung,
- Fig. 2: eine Seitenansicht, teilweise im Längsschnitt eines proximalen Endbereiches der Trokarhülse von Fig. 1 in einer Stellung mit nicht gespreizter Dichtung,
- Fig. 3: eine der Fig. 2 entsprechende Darstellung mit gespreizter Dichtung,
- Fig. 4: eine perspektivische, teilweise aufgeschnittene Seitenansicht des proximalen Endbereiches der Trokarhülse von Fig. 1,
- Fig. 5: eine stirnseitige Ansicht des proximalen Endes der Trokarhülse von Fig. 1 mit einem Teilschnitt im Bereich der Steuerung der Spreizvorrichtung, wobei der mit einem Kreis umrundete Bereich nochmals in stark vergrößertem Umfang dargestellt ist.

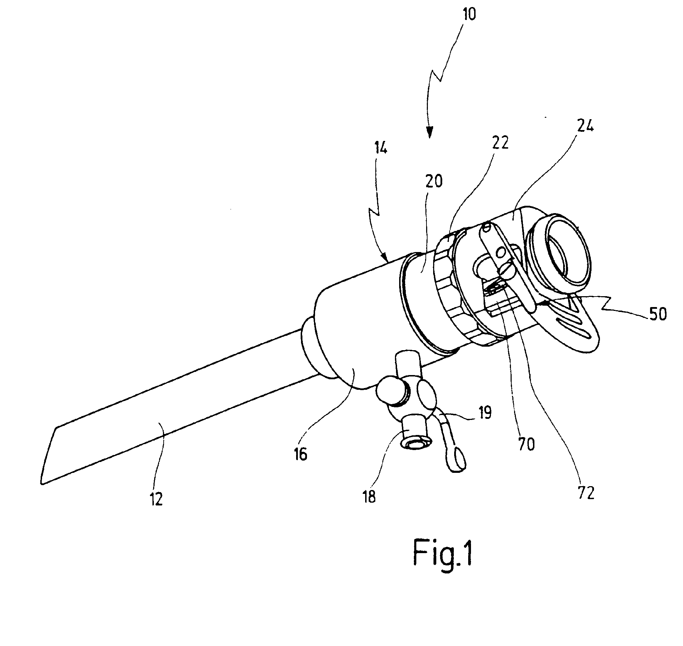

Eine in Fig. 1 dargestellte Trokarhülse ist in ihrer Gesamtheit mit der Bezugsziffer 10 versehen.

Die Trokarhülse 10 weist einen Schaft 12 auf, an dessen proximalseitigem Ende ein Gehäuse 14 angeordnet ist.

Das Gehäuse 14 weist einen äußeren etwa hohlzylindrischen Abschnitt 16 auf, der sich unmittelbar an das proximale Ende des hohlen Schaftes 12 anschließt. Vom äußeren Abschnitt 16 steht radial ein Gasanschluß 18 vor, der über einen Hahn 19 geöffnet und verschlossen werden kann. Über den Gasanschluß 18 kann ein Gas in den Innenraum des Gehäuses 14 und durch den Schaft 12 hindurchgeführt werden.

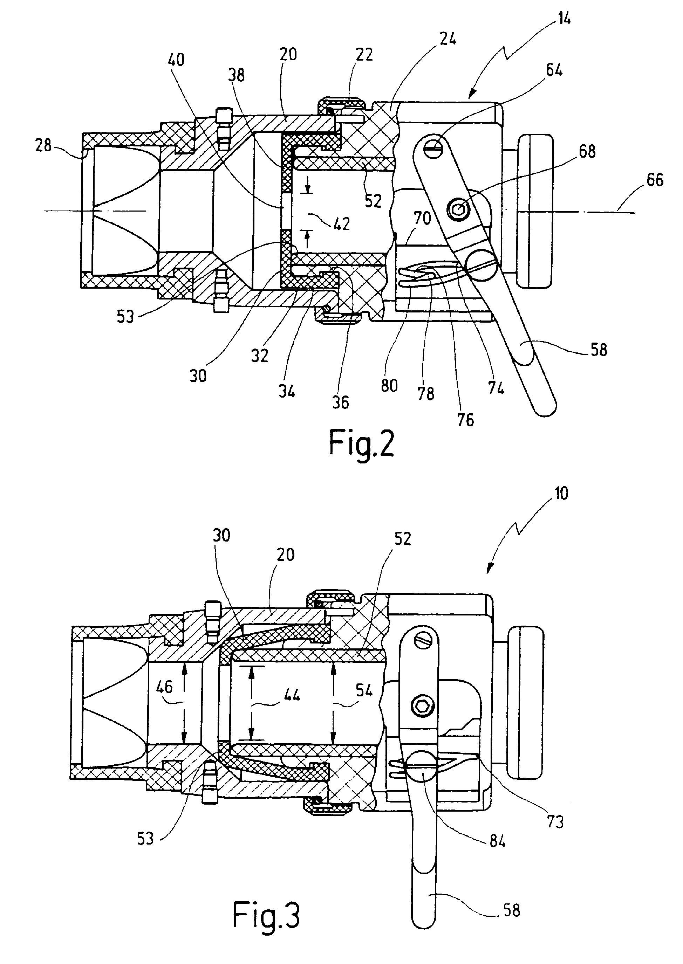

Wie insbesondere aus den Schnittdarstellungen von Fig. 2 und 3 zu ersehen, weist das Gehäuse 14 einen inneren rohrförmigen Abschnitt 20 auf, der von proximal in den äußeren Abschnitt 16 eingeschoben ist. Über eine Überwurfmutter 22 ist an das proximale Ende des inneren Abschnitts 20 ein Endabschnitt 24 befestigt.

Aus der Schnittdarstellung von Fig. 2 ist zu erkennen, daß am distalseitigen Ende des inneren Abschnittes 20 eine Schlitzdichtung 28 befestigt ist, die sich also bis in den äußeren Abschnitt 16 hinein erstreckt.

Diese Schlitzdichtung 28 dient dazu, den Schaft proximalseitig abzudichten.

Diese Dichtung schließt dann gasdicht, wenn, wie in der Schnittdarstellung, kein Instrument oder kein Gegenstand, wie ein Trokardorn oder Obturator, in die Trokarhülse 10 eingeschoben ist.

Proximalseitig der Schlitzdichtung 28 ist eine Dichtung 30 vorgesehen.

Die Dichtung 30 weist einen etwa topfförmigen Körper 32 auf.

Der Körper 32 weist einen Ring 34 oder Rohrabschnitt auf, dessen Achse sich längs der Trokarhülsenachse 66 erstreckt. Am proximalen Ende steht ein nach innen vorstehender Flansch 36 vor. Am gegenüberliegenden Ende ist der Körper 32 mit einem etwa ebenen Boden 38 versehen, der mittig eine Öffnung 40 aufweist. Der Boden 38 erstreckt sich in einer Querschnittsebene senkrecht zur Trokarhülsenachse 66. Die Öffnung 40 weist einen Durchmesser 42 auf, im dargestellten Ausführungsbeispiel 5 mm.

Die Dichtung 30 ist im Bereich ihres ringförmigen Abschnittes 34 und des Flansches 36 zwischen innerem Abschnitt 20 und Endabschnitt 24 eingeklemmt und dort fest gehalten.

Eine Spreizvorrichtung 50 ist nun dazu vorgesehen, um den Durchmesser 42 zu vergrößern, also die Öffnung 40 zu spreizen.

Die Spreizvorrichtung 50 weist eine Hülse 52 auf, deren Außendurchmesser etwa dem lichten Innendurchmesser des Endabschnittes 24 entspricht. Der lichte Innendurchmesser der Hülse 52 ist so groß, daß dieser zumindest dem maximalen lichten Innendurchmesser des Durchganges durch das Gehäuse 14 der Trokarhülse 10 entspricht, wie er in Fig. 3 durch den Durchmesser 46 angedeutet ist.

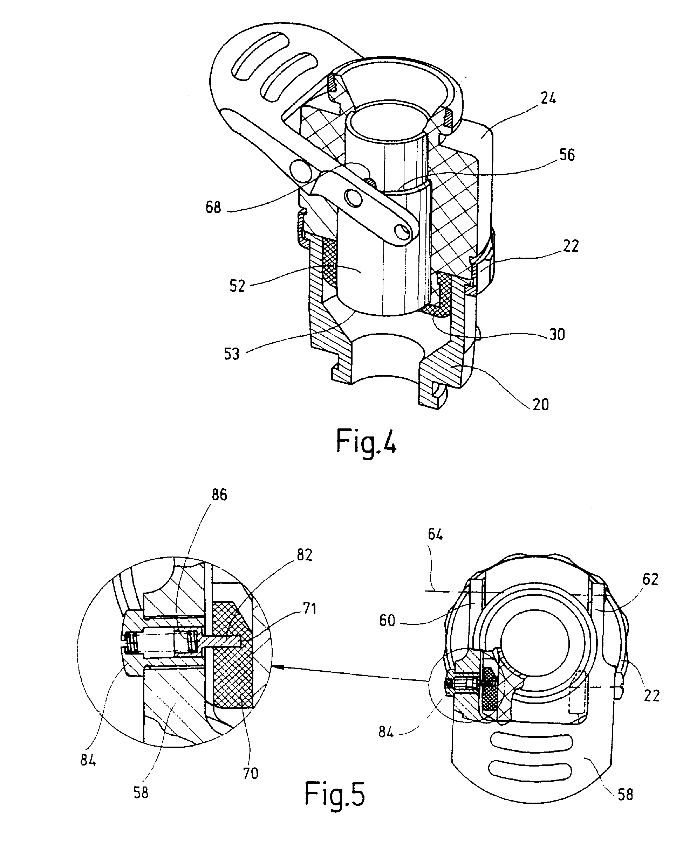

Aus der Schnittdarstellung von Fig. 2 ist zu erkennen, daß eine distalseitige ringförmige Stirnfläche 53 der Hülse 52 auf dem Boden 38 der Dichtung 30 sitzt, und zwar derart, daß diese Stirnfläche 53 die kreisrunde Öffnung 40 koaxial umrundet. An der Außenseite des Endabschnittes 24 ist ein Hebel 58 angeordnet, der, wie insbesondere aus der Draufsicht von Fig. 5 zu erkennen ist, zwei Zinken 60 und 62 aufweist, die sich etwa tangential diametral gegenüberliegend an die Außenseite anlegen. Auf einer der Betätigungsfläche des Hebels 58 gegenüberliegenden Seite sind die Zinken 60 und 62 über Schrauben mit dem Gehäuse 14 verbunden, deren Mittellängsachse eine Schwenkachse 64 des Hebels 58 darstellt.

Beabstandet von der Schwenkachse 64 steht von den Zinken 60 und 62 radial nach innen jeweils ein Zapfen 68 vor, der auf der Ringschulter 56 der Hülse 52 ruht, wie das aus der Darstellung von Fig. 4 ersichtlich ist.

Wird also der Hebel 58 nach distal gedrückt, wie das dem Übergang von Fig. 2 zu Fig. 3 entspricht, schiebt der Zapfen 68 die Hülse 52 nach distal, d.h. diese wird axial längs der Trokarhülse 10 bewegt. Da die Stirnfläche 53 auf dem Boden 38 der Dichtung 30 ruht, wird dabei die Dichtung 30 gedehnt, und dabei wird die Öffnung 40 aufgeweitet, wie das durch den Übergang von Fig. 2 zu Fig. 3 ersichtlich ist.

Die Hülse 52 fährt also in den topfförmigen Körper 32 hinein und dehnt diesen axial, wobei sich die Öffnung 40 im Boden radial aufweitet. Material des Bodens 38 fließt radial nach außen, um die verlängerte bzw. axial gedehnte Topfform zu bilden, dabei spreizt sich die Öffnung 40. Um diesen Materialfluß zu erleichtern, ist die Stirnfläche 53 der Hülse 52 abgerundet.

In Fig. 3 ist eine Stellung dargestellt, in der sich die Dichtung 30 in einer zwischenliegenden Spreizstellung befindet, allerdings kurz vor einer maximalen Spreizstellung. In der in Fig. 3 gezeigten Darstellung hat sich der Durchmesser der Öffnung 40 auf den zwischenliegenden Durchmesser 44 erweitert, im dargestellten Ausführungsbeispiel 10 mm. Durch weiteres Drücken des Hebels 58 nach distal kann die Hülse 52 noch etwas weiter verschoben werden, so daß sich dann die Dichtung 30 noch etwas mehr dehnt und die Öffnung 40 noch etwas weiter aufspreizt, nämlich bis zu dem maximalen Durchmesser 46, im dargestellten Ausführungsbeispiel 11 mm.

Es ist in dem dargestellten Ausführungsbeispiel eine Zwangsführung vorgesehen, durch die die Bewegungen des Hebels 58, also dementsprechend der Hülse 52 und demzufolge die Spreizweiten der Öffnung 40, vorgegeben sind.

Dazu ist an der Außenseite ein Segment in Form eines Kulissensteins 70 vorgesehen, in dem eine Führung 71 in Form einer Herz-Kurve 72 ausgespart ist.

Wie insbesondere aus Fig. 2 zu ersehen, weist die Herz-Kurve 72 einen ersten Abschnitt 74 auf, an den sich ein zurücklaufender kurzer zweiter Abschnitt 76 anschließt. An diesen schließt sich wieder ein vorlaufender dritter Abschnitt 78 an, von dessen äußerem Ende ein vierter Abschnitt 80 wieder zurück zum Ausgangspunkt des ersten Abschnittes 74 führt.

Von der Innenseite der Zinken 60 des Hebels 58 springt, wie das insbesondere aus der Darstellung von Fig. 5 zu ersehen ist, ein Stift 82 vor, der im inneren Hohlraum einer Mutter 84 aufgenommen ist, in der eine vorgespannte Feder sitzt, die den Stift 82 so vorspannt, daß er aus der Mutter herausgedrückt wird.

Der Stift 82 läuft nun in der Herz-Kurve 72, die in dem Kulissenstein 70 ausgeschnitten ist. Die nähere Ausgestaltung soll anhand der Funktionsweise beschrieben werden.

In Fig. 2 ist eine Stellung dargestellt, in der der Hebel 58 in seiner maximal nach proximal verschobenen Stellung ist, also der Stift 82 im Bereich der äußersten Spitze 73 (siehe Fig. 3) der Herz-Kurve 72 zum Liegen kommt.

Dies entspricht der Ruhelage der Dichtung 30, d.h. sie befindet sich in einer Null-Spreizstellung, also nicht gedehnt.

Wird nun der Hebel 58 nach distal gedrückt, bewegt sich der Stift 82 zwangsweise im ersten Abschnitt 74 der Herz-Kurve 72, und er kann so weit vorgeschoben werden, bis das distalseitige Ende dieses ersten Abschnittes 74 erreicht wird. In diesem Zustand ist die Hülse 52 maximal nach distal vorgeschoben, die Dichtung 30 maximal gedehnt, und die Öffnung 40 maximal bis auf den maximalen Durchmesser 46 (z.B. 11 mm) gedehnt.

In diesem maximal gedehnten oder gespreizten Zustand kann nun beispielsweise ein Instrument mit einem Schaftaußendurchmesser von 10 mm durch die Trokarhülse 10 hindurch in den Körper eingeschoben werden. Wird der Hebel 58 freigegeben, bewegt er sich zwangsweise in dem zweiten Abschnitt 76 der Herz-Kurve 72 etwas zurück. Dies wird dadurch erreicht, daß dieser zweite Abschnitt 76 etwas tiefer eingeschnitten ist, und der Stift 82 über die Feder 86 in diese Bahn zwangsweise hineingedrückt wird.

Der Hebel 58 bewegt sich also so weit zurück, bis er in der Mulde des herzförmigen Einschnittes, also am proximalen Ende des zweiten Abschnittes 76, zum Liegen kommt, diese Stellung ist in Fig. 3 dargestellt. Die Hülse 52 ist dabei etwas zurückgefahren, wobei dies durch die Rückstellkraft des elastischen Materials der Dichtung 30 bewerkstelligt wird.

Die Öffnung 40 hat nun die dazwischenliegende Spreizstellung mit dem Öffnungsdurchmesser 44 (10 mm) eingenommen und liegt dann mit ausreichendem Anpreßdruck an der Außenseite des entsprechenden, hier nicht dargestellten Schaftes des eingeschobenen Instrumentes an. Der Hebel 58 verbleibt dann in der in Fig. 3 dargestellten Stellung, ist also in dieser Stellung verrastet.

Bei einem erneuten Drücken auf den Hebel 58 wird dieser von proximal nach distal längs des kurzen dritten Abschnittes 78 der Herz-Kurve 72 bewegt. Auch dies erfolgt wieder zwangsweise dadurch, daß der Stift 82 über die Feder 86 in diese Bahn des dritten Abschnittes 78 hineingedrückt wird. Dadurch wird die Dichtung 30 wieder auf den maximalen Durchmesser 46 gespreizt, so daß nunmehr das Instrument mit dem Außendurchmesser 10 mm einfach von der Trokarhülse 10 abgezogen werden kann. Danach wird der Hebel 58 freigegeben und bewegt sich längs des vierten Abschnittes 80 wieder zum Ausgangspunkt der Herz-Kurve 72, also zu deren Spitze 73, zurück. Diese Bewegung ist wieder zwangsgesteuert, da der Stift 82 von der Feder 86 vom dritten Abschnitt 78 in diesen vierten Abschnitt 80 hineingedrückt wird. Die Rückstellung erfolgt wieder über die Elastizität bzw. die Rückstellkraft des Materials der Dichtung 30, so daß sich dann der Hebel 58 wieder in der in Fig. 2 dargestellten Stellung befindet.

## Patentansprüche

1. Trokarhülse, mit einem proximalseitig angeordneten Gehäuse (14), von dem distalseitig ein Schaft (12) vorspringt, und mit einer Dichtung (30) aus elastischem Material, um ein in die Trokarhülse (10) eingeschobenes Instrument gasdicht nach proximal abzudichten, wobei die Dichtung (30) eine mittige Öffnung (40) aufweist, deren Durchmesser (42, 44, 46) veränderbar ist, wobei die Dichtung (30) eine Ruhelage aufweist, in der die mittige Öffnung (40) einen kleinsten Durchmesser (42) aufweist, und mit einer Spreizvorrichtung (50), die in die Dichtung (30) einfahrbar ist, diese dabei dehnt, wodurch der Durchmesser (44) der mittigen Öffnung (40) vergrößerbar ist, wobei die Spreizvorrichtung (50) eine axial verschiebbare Hülse (52) aufweist, deren eine ringförmige Stirnfläche (53) in die Dichtung (30) einfahrbar ist, **dadurch gekennzeichnet, daß** die Spreizvorrichtung (50) in unterschiedlichen Einfahrstellungen feststellbar ist.

2. Trokarhülse nach Anspruch 1, **dadurch gekennzeichnet, daß** die Dichtung (30) einen sich quer zur Trokarhülsenachse (66) erstreckenden scheibenförmigen Abschnitt (38) aufweist, in dem mittig die Öffnung (40) vorgesehen ist.

3. Trokarhülse nach Anspruch 1, **dadurch gekennzeichnet, daß** die ringförmige Stirnfläche (53) der Hülse koaxial zu einer kreisrunden Öffnung (40) in einem scheibenförmigen Abschnitt der Dichtung (30) angeordnet ist.

4. Trokarhülse nach einem der Ansprüche 1 bis 3, **dadurch gekennzeichnet, daß** die Dichtung (30) einen topfförmigen Körper (32) aufweist, in dessen Boden (38) die Öffnung (40) vorgesehen ist, und daß die Hülse (52) der Spreizvorrichtung (50) in den Körper (32) einfahrbar ist und diesen axial dehnt.

5. Trokarhülse nach Anspruch 4, **dadurch gekennzeichnet, daß** Dichtung (30) und Hülse (52) im Gehäuse (14) angeordnet sind.

6. Trokarhülse nach Anspruch 1, **dadurch gekennzeichnet, daß** eine Führung (71) vorgesehen ist, durch die die Verschiebebewegung der Spreizvorrichtung (50) führbar ist, und daß Rastmittel vorgesehen sind, um die Spreizvorrichtung (50) in bestimmten Verschiebestellungen zu verrasten.

7. Trokarhülse nach Anspruch 6, **dadurch gekennzeichnet, daß** die Führung (71) eine Kulissenführung aufweist, längs der ein Kulissenelement führbar ist, das zwangsweise der Führung (71) folgt.

8. Trokarhülse nach Anspruch 7, **dadurch gekennzeichnet, daß** die Kulissenführung als Herz-Kurve (72) ausgebildet ist, so daß die Spreizvorrichtung (50) zwischen einer Null-Stellung, ohne Spreizung der Dichtung (30), einer maximalen Spreizstellung und einer dazwischenliegenden Spreizstellung bewegbar ist.

9. Trokarhülse nach einem der Ansprüche 1 bis 8, **dadurch gekennzeichnet, daß** die Spreizvorrichtung (50) ein Betätigungsorgan aufweist, das von der Außenseite der Trokarhülse (10) her betätigbar ist.

10. Trokarhülse nach Anspruch 9, **dadurch gekennzeichnet, daß** das Betätigungsorgan als ein um eine quer zur Trokarhülsenachse (66) verlaufende Achse (64) verschwenkbarer Hebel (58) ausgebildet ist.

11. Trokarhülse nach Anspruch 10, **dadurch gekennzeichnet, daß** vom Hebel (58) ein Stift (82) vorspringt, der in der Kulissenführung (71) läuft.

12. Trokarhülse nach Anspruch 11, **dadurch gekennzeichnet, daß** der Stift (82) federbelastet ist.

## Claims

1. Trocar sleeve, comprising at its proximal side a housing (14), from the distal side of which a shaft (12) projects, and with a sealing (30) of elastic material in order to seal an instrument inserted into the trocar sleeve (10) gas-tight into the proximal direction, wherein the sealing (30) comprises a central opening (40), the diameter (42, 44, 46) of which is variable, wherein the sealing (30) comprises a position of rest in which the central opening (40) comprises a smallest diameter (42), and a spreading device (50) which can be inserted into the sealing (30) extending same in doing so, whereby the diameter (44) of the central opening (40) can be enlarged, said spreading device (50) comprises a sleeve (52) which can be moved axially, a ring-shaped facial end (53) thereof can be introduced into the sealing (30), **characterized in that** the spreading device (50) can be locked in different displacing positions.

2. Trocar sleeve of claim 1, **characterized in that** the sealing (30) has a disk-like-section (38) extending transverse to the axis (66) of the trocar sleeve, the opening (40) is provided centrally within that section (38).

3. Trocar sleeve of claim 1, **characterized in that** the ring-shaped facial end (53) of the sleeve is arranged coaxially to the circular opening (40) in the disk-like-section of the sealing (30).

4. Trocar sleeve of anyone of claims 1 through 3, **characterized in that** the sealing (30) has a pot-shaped body (32), the opening (40) being provided in a bottom (38) thereof, and **in that** the sleeve (52) of the spreading device can be inserted into the body (32), thereby stretching it axially.

5. Trocar sleeve of claim 4, **characterized in that** the sealing (30) and the sleeve (52) are arranged within the housing (14).

6. Trocar sleeve of claim 1, **characterized in that** a guide (71) is provided for guiding the displacing movement of the spreading device. (50), and **in that** locking means are provided for interlocking the spreading device (50) in different displacing positions.

7. Trocar sleeve of claim 6, **characterized in that** the guide (71) has a slot-link guide, a slidable member is guided within that guide (71) compulsory.

8. Trocar sleeve of claim 7, **characterized in that** the slot-link guide is designed as a heart curve (72) guiding said spreading device (50) between a zero position without spreading the sealing (30), a maximum stretching position and an intermediate position.

9. Trocar sleeve of anyone of claims 1 through 8, **characterized in that** the spreading device (50) has an actuation member which can be activated from an outer side of the trocar sleeve (10).

10. Trocar sleeve of claim 9, **characterized in that** said actuation member is configured as a lever (58) being pivotable about an axis (64) transversally to said longitudinally extending trocar sleeve axis (66).

11. Trocar sleeve of claim 10, **characterized in that** a pin (82) projects from said lever (58), which pin (82) is guided within said slot-link guide (71).

12. Trocar sleeve of claim 11, **characterized in that** the pin (82) is forced by a spring.

## Revendications

1. Douille de trocart, avec un boîtier (14) disposé côté proximal, duquel dépasse une tige (12) côté distal, et avec un joint (30) à base de matériau élastique, prévu pour d'étanchéifier un instrument introduit dans la douille de trocart (10) de façon étanche au gaz vers le côté proximal, le joint (30) présentant une ouverture (40) centrée, dont le diamètre (42, 44, 46) peut être modifié, le joint (30) présentant une position de repos, dans laquelle l'ouverture (40) centrée présente un diamètre (42) minimal, et avec un dispositif d'écartement (50), qui peut être entré dans le joint (30), celui-ci se dilatant à cette occasion, de sorte que le diamètre (44) de l'ouverture (40) centrée peut être agrandi, le dispositif d'écartement (50) présentant une douille (52) coulissant axialement, dont une face frontale (53) de forme annulaire peut entrer dans le joint (30), **caractérisée en ce que** le dispositif d'écartement (50) peut être immobilisé dans différentes positions d'entrée.

2. Douille de trocart selon la revendication 1, **caractérisée en ce que** le joint (30) présente un tronçon (38) en forme de disque s'étendant transversalement à l'axe (66) de la douille de trocart, dans lequel l'ouverture (40) est prévue au centre.

3. Douille de trocart selon la revendication 1, **caractérisée en ce que** la surface frontale (53) de forme annulaire de la douille est disposée de façon coaxiale par rapport à une ouverture (40) circulaire dans un tronçon en forme de disque du joint (30).

4. Douille de trocart selon l'une des revendications 1 à 3, **caractérisée en ce que** le joint (30) présente un corps (32) en forme de pot, dans le fond (38) duquel est prévue l'ouverture (40), et **en ce que** la douille (52) du dispositif d'écartement (50) peut entrer dans le corps (32) et dilate celui-ci axialement.

5. Douille de trocart selon la revendication 4, **caractérisée en ce que** le joint (30) et la douille (52) sont disposés dans le boîtier (14).

6. Douille de trocart selon la revendication 1, **caractérisée en ce qu'**il est prévu un guidage (71), par lequel le mouvement de coulissement du dispositif d'écartement (50) peut être guidé et **en ce qu'**il est prévu des moyens d'encliquetage afin d'encliqueter le dispositif d'écartement (50) dans certaines positions de coulissement.

7. Douille de trocart selon la revendication 6, **caractérisée en ce que** le guidage (71) présente un guide à coulisse, le long duquel un élément à coulisse peut être guidé, lequel suit de façon forcée le guide (71).

8. Douille de trocart selon la revendication 7, **caractérisée en ce que** le guide à coulisse est conçu comme une cardioïde (72), de sorte que le dispositif d'écartement (50) peut être déplacé entre une position zéro, sans écartement du joint (30), une position d'écartement maximale et une position d'écartement intermédiaire.

9. Douille de trocart selon l'une quelconque des revendications 1 à 8, **caractérisée en ce que** le dispositif d'écartement (50) présente un organe d'actionnement qui peut être actionné à partir du côté extérieur de la douille de trocart (10).

10. Douille de trocart selon la revendication 9, **caractérisée en ce que** l'organe d'actionnement est conçu comme un levier (58) pouvant basculer autour d'un axe (64) disposé transversalement à l'axe (66) de la douille de trocart.

11. Douille de trocart selon la revendication 10, **caractérisée en ce qu'**une goupille (82), qui se déplace dans le guide à coulisse (71), dépasse du levier (58).

12. Douille de trocart selon la revendication 11, **caractérisée en ce que** la goupille (82) est sollicitée par un ressort.
